# EUROPEAN PATENT APPLICATION

(11) **EP 1 153 737 A2**
(43) Date of publication of application: **14.11.2001**
(21) Application number: 01303686.8
(22) Date of filing: 23.04.2001
(51) Int. Cl.: B32B 5/26, D04H 13/00, A61F 13/15

(54) **Breathable and liquid-impervious composite sheet**

(30) Priority: 09.05.2000 JP 2000135981
(71) Applicant: UNI-CHARM CORPORATION, Kawanoe-shi Ehime-ken (JP)
(72) Inventor: Matsushita, Michiyo, Technical Center, Mitoyo-gun, Kagawaken-769-1502 (JP); Atsuta, Yasushi, Technical Center, Mitoyo-gun, Kagawaken-769-1502 (JP)
(74) Representative: Parry, Christopher Stephen

(57) **Abstract**

A breathable and liquid-impervious composite sheet 1 includes a flexible, breathable and liquid-impervious thermoplastic synthetic resin film 2 and heat-sealable nonwoven fabric layers 3A, 3B placed upon opposite surface of the film 2. The film 2 has a basis weight of 0.098 - 0.392 N/m² and a thermal shrink percentage of 3 - 18 % under 60 °C thermal atmosphere while the nonwoven fabric layers 3A, 3B have a basis weight of 0.049 - 0.294 N/m². The sheet 1 is formed with plural arrays of heat-sealed regions 4 each extending in transverse direction Y and spaced one from another in longitudinal direction X intersecting the transverse direction Y. Between each pair of the adjacent heat-sealed regions 4, the nonwoven fabric layers 3A, 3B describe circular arcs which are convex outward in a thickness direction of the sheet 1 from the opposite surfaces of the film 2.

## Description

This invention relates to a breathable and liquid-impervious composite sheet suitable for use in a disposable wearing article such as an outer gown for surgeon or a patient or nightgown for patient.

Japanese Patent Application Publication No. 1998-295724A describes a three-layered breathable and liquid-impervious composite sheet comprising a third fibrous layer of meltblown-nonwoven fabric, and first and second fibrous layers of spunbond- or spunlace-nonwoven fabric and placed upon opposite surfaces of the third fibrous layer. This composite sheet of prior art is embossed by a heat-embossing roll so that the first, second and third fibrous layers are intermittently heat-sealed in heat-sealing regions. This composite sheet of prior art has high flexibility and surface friction strength.

The composite sheet disclosed in the Publication has its sheet surfaces being substantially flat and can not be compressively deformed inward in a thickness direction of the sheet enough to ensure a desired elasticity even when compressed in its thickness direction. To improve the elasticity of the sheet, it may be contemplated to form the first and second fibrous layers with a plurality of high density projections extending outward in the thickness direction of the sheet. However, such high density projections on the first and second fibrous layers inevitably increase the basis weight of these fibrous layers and deteriorate the flexibility of the composite sheet.

It is an object of this invention to provide a breathable and liquid-impervious composite sheet having high elasticity and flexibility.

According to this invention, there is provided the breathable and liquid-impervious composite sheet including flexible, breathable and liquid-impervious thermoplastic synthetic resin film and heat-sealable fibrous nonwoven fabric layers placed upon opposite surfaces of the synthetic resin film, and obtained by heat-sealing the synthetic resin film and the fibrous nonwoven fabric layers together in the course of a heat treatment process including an embossing roll.

The improvement according to this invention is in that the synthetic resin film has a basis weight of 0.098 - 0.392 N/m² arid a thermal shrink percentage of 3 - 18 % under 60 °C (± 5 °C) thermal atmosphere while the nonwoven fabric layers have a basis weight of 0.049 - 0.294 N/m², wherein the sheet is formed with plural arrays of heat-sealed regions each extending in transverse direction of the composite sheet and spaced one from another in longitudinal direction intersecting the transverse direction and wherein, between each pair of the adjacent heat-sealed regions, the nonwoven fabric layers describe circular arcs which are convex outward in a thickness direction of the sheet from the opposite surfaces of the synthetic resin film.

According to one preferred embodiment of this invention, the heat-sealed regions are arranged intermittently with spacings each of a given length in the transverse direction so that a length of each the heat-sealed region and a length of each the spacing in the transverse direction are in a relationship of the length of the heat-sealed region > the length of the spacing and wherein the spacings in one array of the heat-sealed regions are not aligned with the spacings in the array of heat-sealed region which is adjacent to the one array in the longitudinal direction of the composite sheet while the respective spacings in the one array of heat-sealed regions are aligned with the respective heat-sealed regions in the array which is adjacent to the one array so that, as viewed in the longitudinal direction, respective the heat-sealed regions as well as respective the spacings in these arrays are in a staggered relationship.

According to another preferred embodiment of this invention, the composite sheet 1 has a relative hardness of 40 - 70 mm in the longitudinal direction as measured by the cantilever test.

According to still another preferred embodiment of this invention, the composite sheet has a compressive modulus of 70 - 95% in its region except the heat-sealed regions.
Fig. 1 is a perspective view of a composite sheet;
Fig. 2 is a sectional view of the composite sheet taken along line A - A in Fig. 1;
Fig. 3 is a diagram illustrating relative positions of heat-sealed regions in the composite sheet;
Fig. 4 is a diagram illustrating an example of the process for making the composite sheet shown in Fig. 1; and
Fig. 5 is a diagram illustrating another example of the process for making the composite sheet shown in Fig. 1.

Details of the breathable and liquid-impervious composite sheet according to this invention will be more fully understood from the description given hereunder with reference to the accompanying drawings.

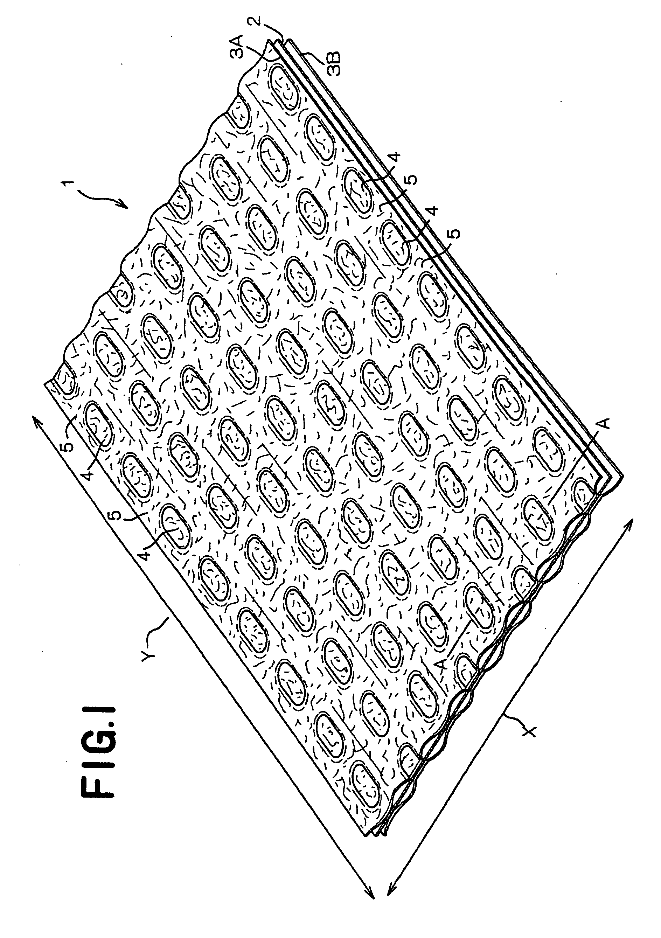

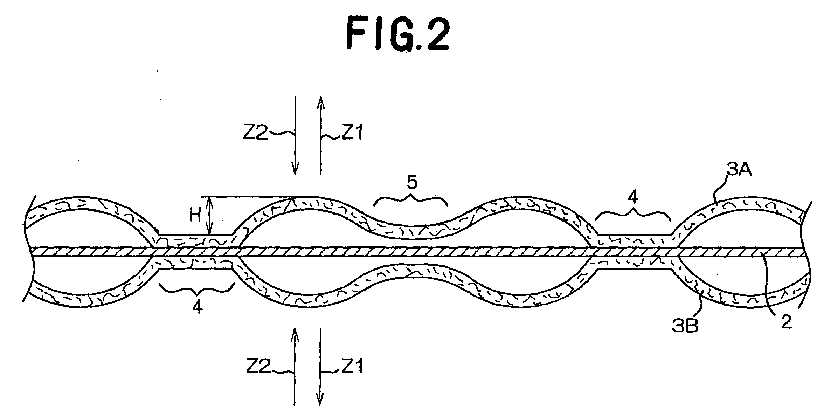

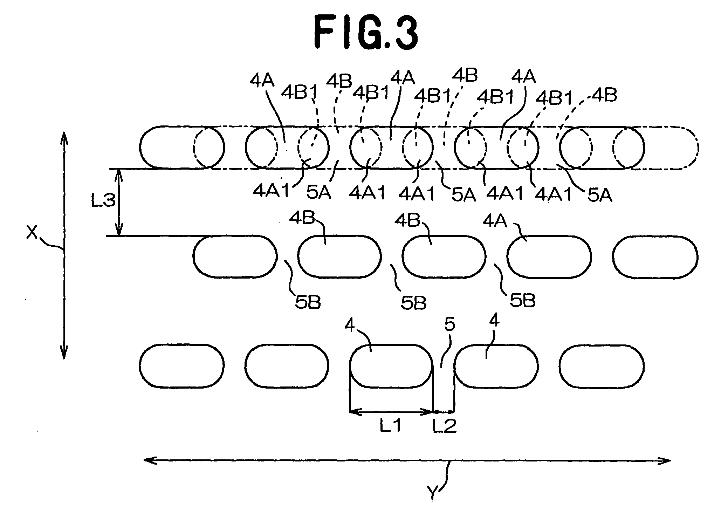

Fig. 1 is a perspective view of a composite sheet 1, Fig. 2 is a sectional view of the composite sheet 1 taken along line A - A in Fig. 1 and Fig. 3 is a diagram illustrating relative positions of heat-sealed regions 4 in the composite sheet 1. The composite sheet 1 is of a three-layered structure comprising a layer of flexible, breathable and liquid-impervious thermoplastic synthetic resin film 2 and two nonwoven fabric layers 3A, 3B each made of thermoplastic synthetic resin fiber and placed on opposite surfaces of the film 2, respectively.

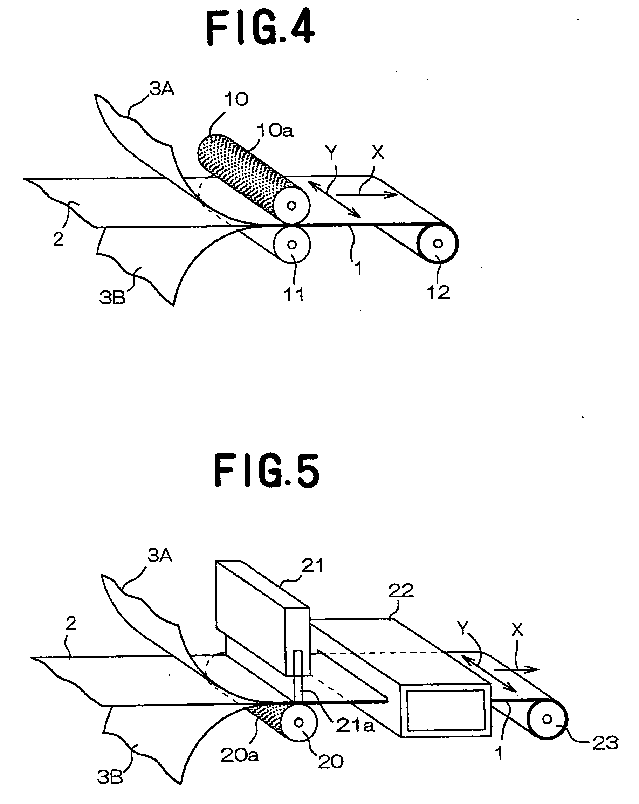

In the course of a heat treatment process (See Figs. 4 and 5) including embossing rolls 10, 20, the synthetic resin film 2 and nonwoven fabric layers 3A, 3B are partially heat-sealed together to form the composite sheet 1. The synthetic resin film 2 is thermally shrinkable in longitudinal direction X and transverse direction Y of the composite sheet 1, at least in the longitudinal direction X. The direction referred to herein as the longitudinal direction X should be understood to be the direction extending in parallel to the circumferential direction defined by a peripheral surfaces of the embossing rolls 10, 20 as will be described later. The direction referred to herein as the transverse direction Y should be understood to be the direction extending transversely of the circumferential direction defined by the peripheral surfaces of the embossing rolls 10, 20.

The composite sheet 1 is formed with a plurality of heat-sealed regions 4 arranged intermittently with spacings 5 each of a given length L2 in the transverse direction Y of the composite sheet 1. Each array of these heat-sealed regions 4 is spaced apart from the adjacent array of the heat-sealed regions 4 in the longitudinal direction X of the composite sheet 1.

As will be apparent from Fig. 3, a length L1 of each heat-sealed region 4 in the transverse direction Y is larger than the length L2 of each spacing in the direction Y, i.e., the length of the heat-sealed region > the length of the spacing. The spacings 5A in one array of the heat-sealed region 4A are not aligned with the spacings 5B in the array of heat-sealed region 4B which is adjacent to the one array in the longitudinal direction X of the composite sheet 1. Specifically, the respective spacings 5A in the one array of heat-sealed region array 4A are aligned with the respective heat-sealed regions 4B in the array which is adjacent to the one array. As viewed in the longitudinal direction X, the respective heat-sealed regions 4A, 4B as well as the respective spacings 5A, 5B in these arrays are in a staggered relationship. As indicated by imaginary lines in Fig. 3, the heat-sealed regions 4A, 4b in each pair of the arrays being adjacent to each other in the longitudinal direction X are arranged so that transversely opposite ends 4A1 of the heat-sealed region 4A overlap transversely opposite ends 4B1 of the heat-sealed region 4B.

Between each pair of the adjacent heat-sealed regions 4, the nonwoven fabric layers 3A, 3B describe circular arcs which are convex outwardly in a thickness direction of the composite sheet 1, i.e., in the direction Z1. In each spacing 5, the nonwoven fabric layers 3A, 3B are slightly spaced apart from the synthetic resin film 2 outward of the thickness direction, i.e., in the direction Z1, and extend substantially in parallel to the synthetic resin film 2.

When a pressure is exerted inward in the thickness direction, i.e., in direction Z2, upon the composite sheet 1, the portions of the nonwoven fabric layers 3A, 3B extending between each pair of the adjacent heat-sealed regions 4 are compressively deformed against this pressure. Being relieved of this pressure, the nonwoven fabric layers 3A, 3B are spaced apart again from opposite surfaces of the synthetic resin film 2 so as to describe the circular arcs which are convex outward in the thickness direction, i.e., in the direction Z1.

The composite sheet 1 has a relative hardness of 40 ∼ 70 mm in the longitudinal direction as measured by the cantilever test and a compressive modulus of 70 ∼ 95 % in the region except the heat-sealed regions 4. Measurement of these relative hardness and the compressive modulus was conducted in according to JIS: L1096. The relative hardness exceeding 70 mm and the compressive modulus exceeding 95 % would unacceptably deteriorate a flexibility of the composite sheet 1. The relative hardness less than 40 mm and the compressive modulus less than 70 % would unacceptably deteriorate an elasticity of the composite sheet 1.

A dimension L3 by which each pair of the heat-sealed regions being adjacent in the longitudinal direction X are spaced apart from each other is preferably in a range of 1.5 ∼ 7.0 mm. The dimension L3 less than 1.5 mm would lead to formation of an excessive number of the heat-sealed regions 4. Consequently, the relative hardness of the composite sheet 1 would exceed the preferable range and the flexibility of the composite sheet 1 would be correspondingly deteriorated. The dimension L3 exceeding 7.0 mm, on the other hand, would unacceptably decrease the number of the heat-sealed regions 4 and peel strength as well as shape stability of the composite sheet 1 would be correspondingly deteriorated.

The synthetic resin film 2 preferably has a basis weight of 0.098 ∼ 0.392 N/m² and a thickness of 20∼30 *µ*m. The nonwoven fabric layers 3A, 3B preferably have a basis weight of 0.049 ∼ 0.294 N/m². It is assumed that the synthetic resin film has a basis weight less than 0.098 N/m² and a thickness less than 20 *µ*m while the nonwoven fabric layers 3A, 3B have a basis weight of 0.049 N/m². In this case, both the synthetic resin film 2 and the nonwoven fabric layers 3A, 3B would have strength deteriorated and it is apprehended that troubles such as breakage and/or pinhole formation might occur in the heat-sealed regions 4 as the film 2 and nonwoven fabric layers 3A, 3B are heat-sealed together. Assume, on the other hand, that the synthetic resin film 2 has a basis weight exceeding 0.392 N/m² and a thickness exceeding 30 *µ*m while the nonwoven fabric layers 3A, 3B have a basis weight exceeding 0.294 N/m², the composite sheet 1 would have a relative hardness exceeding the range and a flexibility correspondingly deteriorated.

The length L1 of the heat-sealed region 4 in the transverse direction Y is preferably in a range of 1.50 ∼ 3.00 mm and the length L2 of the spacing 5 in the transverse direction Y is preferably 1.00 ∼ 1.20 mm. Assumed that the length L1 is less than 1.50 mm and the length L2 exceeds 1.20 mm, the composite sheet 1 would have its peel strength and shape stability unacceptably deteriorated.

Between each pair of the adjacent heat-sealed regions 4, the nonwoven fabric layers 3A, 3B preferably have a height H of 0.05 ~ 0.20 mm as measured from their bottoms to their tops. The height H less than 0.05 mm would prevent the nonwoven fabric layers 3A, 3B from being compressively deformed sufficiently and the compressive modulus of the composite sheet 1 would be less than the range. Correspondingly, the composite sheet 1 could not have a desired elasticity. The height H exceeding 0.20 mm would increase a bulkiness of the composite sheet 1 and correspondingly increase a frictional drag of the composite sheet's surface.

Fig. 4 is a diagram illustrating an example of the process for making the composite sheet 1 (shown in Fig. 1) and Fig. 5 is a diagram illustrating another example of the process for making the composite sheet 1 (shown in Fig. 1).

The process illustrated in Fig. 4 includes an embossing roll 10 and a crown roll 11 opposed to the embossing roll 10 and a take-up roll 12 provided at downstream of these rolls 10, 11 to take up the finished composite sheet 1. The embossing roll 10 and the crown roll 11 are rotated at an identical speed. The take-up roll 12 is rotated at a speed slightly lower than the speed at which the embossing roll 10 and crown roll 11 are rotated.

The embossing roll 10 is formed on its peripheral surface with a plurality of projections 10a and the crown roll 11 has a flat peripheral surface. The embossing roll 10 and the crown roll 11 are heated at a temperature of approximately 80 °C-approximately 110°C. Though not illustrated in details, the projections 10a on the embossing roll 10 are arranged intermittently in the direction (i.e., transverse direction Y) transversely of the circumferential direction of the embossing roll 10 in a plurality of arrays. The projections 10a in one array are aligned with the spacings in the array adjacent to the one array in the circumferential direction. In other words, the projections 10a in each pair of the adjacent array are in staggered relationship.

These rolls 10, 11 cooperate so that the projections 10a of the embossing roll 10 come in contact with the peripheral surface of the crown roll 11. Flat surface of the embossing roll 10 except the projections 10a and the flat surface of the crown roll 11 keep a small clearance therebetween during cooperation of these rolls 10, 11.

According to this process, the synthetic resin film 2 being continuous in the longitudinal direction X and the nonwoven fabric layers 3A, 3B both being continuous in the longitudinal direction X are fed into a nip defined between the embossing roll 10 and the crown roll 11 so the nonwoven fabric layers 3A, 3B may be placed upon opposite surface of the synthetic resin film 2, respectively. The synthetic resin film 2 and the nonwoven fabric layers 3A, 3B are heat-sealed together by the projections 10a of the embossing roll 10 and the peripheral surface of the crown roll 11 coming in contact with each other.

While the synthetic resin film 2 and the nonwoven fabric layers 3A, 3B are not heat-sealed together between the flat surface portion of the roll 10 and the flat peripheral surface of the crown roll 11, it should be understood that the synthetic resin film 2 is heated via the nonwoven fabric layers 3A, 3B directly heated by the rolls 10, 11 and thereby thermally shrunk at the shrinkage percentage.

In the course of this process, the thermal shrinkage of the synthetic resin film 2 causes the heat-sealed regions 4 get near one to another in the longitudinal direction X immediately after the synthetic resin film 2 and the nonwoven fabric layers 3A, 3B have been heat-sealed together and discharged from the nip of the rolls 10, 11. Between each pair of the adjacent heat-sealed regions 4, the nonwoven fabric layers 3A, 3B are not thermally shrinkable in spite of being heated by the rolls 10, 11. In consequence, the nonwoven fabric layers 3A, 3B describe a circular arc which is convex upward in the thickness direction of the composite sheet 1, i.e., in the direction Z1 between each pair of the adjacent heat-sealed regions 4 as the dimension L3 between these adjacent heat-sealed regions 4 is thermally shrunk.

The composite sheet 1 discharged from the nip between the embossing roll 10 and the crown roll 11 is cooled by heat dissipation and taken up on the take-up roll 12. Rotary speed of the take-up roll 12 is lower than that of the embossing roll 10 and the crown roll 11 and therefore it is not apprehended that the thermally shrunk synthetic resin film 2 might be stretched by the take-up roll 12 in the longitudinal direction X. The synthetic resin film 2 ceases to be thermally shrunk as it is cooled and is maintained in its shrunk condition.

The another example of the process illustrated in Fig. 5 will be now described. This process includes an embossing roll 20 adapted to be rotated at a predetermined speed, an ultrasonic sealer 21, a heater 22 provided at downstream of the roll 20 to heat the composite sheet 1 and a take-up roll 23 provided at downstream of the heater 22 to take up the finished composite sheet 1. The take-up roll 23 is rotated at a speed slightly lower than the speed at which the embossing roll 20 is rotated.

The embossing roll 20 is formed on its peripheral surface with a plurality of projections 20a. The projections 20a on the embossing roll 20 are arranged with spacings each having a predetermined spacing intermittently in the direction (i.e., transverse direction Y) transversely of the circumferential direction of the embossing roll 20 in a plurality of arrays. The projections 20a in one array are aligned with the spacings in the array adjacent to the one array in the circumferential direction (i.e., the longitudinal direction X) In other words, the projections 10a in each pair of the adjacent array are in staggered relationship. An anvil 21a of the ultrasonic sealer 21 is adapted to come in contact with the projections 20a on the peripheral surface of the roll 20.

According to this process, the synthetic resin film 2 being continuous in the longitudinal direction X and the nonwoven fabric layers 3A, 3B both being continuous in the longitudinal direction X are fed into a nip defined between the embossing roll 20 and the ultrasonic sealer 21 so that the nonwoven fabric layers 3A, 3B may be placed upon opposite surface of the synthetic resin film 2, respectively. The synthetic resin film 2 and the nonwoven fabric layers 3A, 3B are heat-sealed together by the projections 20a of the embossing roll 20 and the anvil 21a of the ultrasonic sealer 21 coming in contact with each other.

The composite sheet 1 comprising the synthetic resin film 2 and the nonwoven fabric layers 3A, 3B heat-sealed together in this manner is then fed to the heater 22. In the heater 22, the composite sheet is subjected to hot blast at a temperature of approximately 100 °C - approximately 150 °C and thermally shrunk at the shrink percentage.

In the heater 22, the thermal shrinkage of the synthetic resin film 2 causes the heat-sealed regions 4 get near one to another in the longitudinal direction X. Between each pair of the adjacent heat-sealed regions 4, the nonwoven fabric layers 3A, 3B are not thermally shrinkable in spite of being heated by the heater 22. In consequence, the nonwoven fabric layers 3A, 3B describe a circular arc which is convex outward in the thickness direction of the composite sheet 1, i.e., in the direction Z1, between each pair of the adjacent heat-sealed regions 4 as the dimension L3 between these adjacent heat-sealed regions 4 is thermally shrunk.

The composite sheet 1 discharged from the heater 22 is cooled by heat dissipation and taken up on the take-up roll 23. The synthetic resin film 2 ceases to be thermally shrunk as it is cooled and is maintained in its shrunk condition.

A thermal shrink percentage per unit area of the synthetic resin film 2 is in a range of 3 ∼ 18 %, preferably in a range of 5 ∼ 13 % under a thermal atmosphere at 60 °C (± 5 °C). It is assumed now that the shrink percentage of the synthetic resin film 2 is less than 3 %. In this case, even if the synthetic resin film 2 is thermally shrunk to reduce the dimension L3 between each pair of the adjacent heat-sealed regions 4, the nonwoven fabric layers 3A, 3B could not sufficiently curved toward the direction Z1 to ensure an elasticity desired for the composite sheet 1. It is assumed, on the other hand, that the shrink percentage exceeds 18 %. In this case, the respective heat-sealed regions 4 would come too close to one another due to thermal shrinkage of the synthetic resin film 2. Consequently, a substantially excessive number of the heat-sealed regions 4 would be formed on the composite sheet 1 and the composite sheet 1 would have its flexibility unacceptably deteriorated.

The synthetic resin film 2 may be made of low density polyethylene, linear low density polyethylene, intermediate density polyethylene or metallocene polyethylene. The nonwoven fabric layers 3A, 3B may be formed with a nonwoven fabric of polyethylene fiber having the same composition as the synthetic resin film 2, for example, of spunbond-, spunlace-, needlepunch-, meltblown-, thermalbond- or chemicalbond-type. Component fiber of these nonwoven fabric layers 3A, 3B may be selected from a group of core-sheath-type conjugated fiber of which the sheath is formed with polyethylene having the same composition as the synthetic resin film 2 and the core is formed with any one of polypropylene, polyester and polyethylene terephthalate. The synthetic resin film 2 and the nonwoven fabric layers 3A, 3B may be formed with polyethylene being the same in composition as well as in melting point to facilitate these film 2 and nonwoven fabric layers 3A, 3B to be heat-sealed together.

It is also possible to use block-polymerized polypropylene, random-polymerized polypropylene, homopolymerized polypropylene or metallocene polypropylene as material for the synthetic resin film 2. In combination with the synthetic resin film 2 made of polypropylene, the nonwoven fabric layers 3A, 3B may be formed with polypropylene fiber of the same composition as the synthetic resin film 2.

The heat-sealed regions 4 have been described above with reference to the accompanying drawings to be arranged-intermittently in the transverse direction Y of the composite sheet 1. However, it is also possible to arrange these heat-sealed regions 4 so that they extend continuously in the transverse direction Y of the composite sheet 1.

The composite sheet 1 according to this invention is suitably used as the stock material for a disposable wearing article such as an outer gown for surgeon or a patient or nightgown for patient. Such composite sheet 1 is applicable also as the stock material for a backsheet in a disposable diaper, a diaper cover, training pants or incontinent pants.

The breathable and liquid-impervious composite sheet proposed by this invention comprises the flexible, breathable and liquid-impervious synthetic resin film and the nonwoven fabric layers placed upon opposite surfaces of. the synthetic resin film, respectively. The synthetic resin film has a thermal shrink percentage of 3 ∼ 18 % per unit area under 60 °C (± 5 °C) thermal atmosphere and, between each pair of the adjacent heat-sealed regions, the nonwoven fabric layers describe circular arcs which are convex outward in the thickness direction of the composite sheet from the opposite surfaces of the synthetic resin film. Under pressure exerted upon the composite sheet inward in the thickness direction, the respective nonwoven fabric layers are compressively deformed against the pressure. However, once relieved of the pressure, the nonwoven fabric layers restore their initial circular arcs which are convex outward in the thickness-direction from the opposite surfaces of the synthetic resin film.

The composite sheet has a relative hardness of 40 ∼ 70 mm in the longitudinal direction and a compressive modulus of 70 ∼ 95 % between each pair of the adjacent heat-sealed regions. In the composite sheet, the synthetic resin film has a basis weight of 0.098 - 0.392 N/m² and the nonwoven fabric layers have a basis weight of 0.049 ∼ 0.294 N/m². Accordingly, the composite sheet has a thickness appropriately limited so as to ensure high elasticity and flexibility.

## Claims

1. A breathable and liquid-impervious composite sheet comprising a flexible, breathable and liquid-impervious thermoplastic synthetic resin film and heat-sealable fibrous nonwoven fabric layers placed upon opposite surfaces of said synthetic resin film, and obtained by heat-sealing said synthetic resin film and said fibrous nonwoven fabric layers together in the course of a heat treatment process including an embossing roll, wherein:
said synthetic resin film has a basis weight of 0.098 ∼ 0.392 N/m² and a thermal shrink percentage of 3 - 18 % under 60 °C (± 5 °C)thermal atmosphere while said nonwoven fabric layers have a basis weight of 0.049 ∼ 0.294 N/m², wherein the sheet is formed with plural arrays of heat-sealed regions 4 each extending in transverse direction of said composite sheet and spaced one from another in longitudinal direction intersecting the transverse direction and wherein, between each pair of the adjacent heat-sealed regions, the nonwoven fabric layers describe circular arcs which are convex outward in a thickness direction of the sheet from the opposite surfaces of said synthetic resin film.

2. The composite sheet according to Claim 1, wherein said heat-sealed regions are arranged intermittently with spacings each of a given length in said transverse direction so that a length of each said heat-sealed region and a length of each said spacing in said transverse direction are in a relationship of the length of the heat-sealed region > the length of the spacing and wherein said spacings in one array of said heat-sealed regions are not aligned with the spacings in the array of heat-sealed region which is adjacent to said one array in the longitudinal direction of the composite sheet while the respective spacings in said one array of heat-sealed regions are aligned with the respective heat-sealed regions in the array which is adjacent to said one array so that, as viewed in said longitudinal direction, respective said heat-sealed regions as well as respective said spacings in these arrays are in a staggered relationship.

3. The composite sheet according to Claim 1, wherein said composite sheet 1 has a relative hardness of 40 ∼ 70 mm in said longitudinal direction as measured by the cantilever test.

4. The composite sheet according to Claim 1, wherein said composite sheet has a compressive modulus of 70 ∼ 95% in its region except said heat-sealed regions.
